# EUROPÄISCHE PATENTSCHRIFT

(11) **EP 2 253 307 B1**
(45) Veröffentlichungstag und Bekanntmachung des Hinweises auf die Patenterteilung: **08.07.2015**
(21) Anmeldenummer: 10004937.8
(22) Anmeldetag: 02.05.2007
(51) Int. Cl.: A61K 9/00, A61K 31/205, A61K 33/06, A61K 31/198, A23L 1/29, A61K 9/14

(54) **L-Carnitin zur Kristallisationshemmung**
L-Carnitin for supression of crystallisation
L-carnitine pour la supression de la cristallisation

(30) Priorität: 12.05.2006 EP 06009865
(43) Veröffentlichungstag der Anmeldung: 24.11.2010
(62) Teilanmeldung aus: 07008906.5
(73) Patentinhaber: Verla-Pharm Arzneimittelfabrik Apotheker H.J. von Ehrlich GmbH & Co. KG, D-82327 Tutzing (DE)
(72) Erfinder: Hopf, Günter, 82327 Tutzing (DE)
(74) Vertreter: Müller-Boré & Partner Patentanwälte PartG mbB

(56) Entgegenhaltungen:
- EP-A1- 1 163 904
- EP-A2- 0 402 755
- EP-A2- 0 680 945
- WO-A-01/52647
- WO-A-01/91759
- US-A1- 2002 132 219

## Beschreibung

Die vorliegende Erfindung betrifft lagerstabile wässrige bzw. gelartige Zusammensetzungen, umfassend mindestens eine Magnesiumverbindung, ausgewählt aus einem Magnesiumsalz oder einer Magnesiumkomplexverbindung, und L-Carnitin, und deren Verwendung zur Herstellung eines Arzneimittels zur Unterstützung des Stoffwechsels, insbesondere im Muskelgewebe, und des Muskelaufbaus bzw. deren Verwendung als Nahrungsergänzungsmittel oder im Veterinärbereich als Tierfutterzusatz, insbesondere für Pferde, Geflügel, Tauben, Schweine, Rinder, Schafe und Kamele.

Im Stand der Technik sind wässrige Magnesium-enthaltende Lösungen (beispielsweise Nupafeed®, vertrieben von Verla-Pharm) bekannt, die etwa 50 Gew.-% Magnesiumaspartathydrochlorid enthalten. Diese und höhere Magnesiumkonzentrationen sind bei derartigen Lösungen aufgrund der zunehmenden Kristallisationsneigung der enthaltenen Magnesiumverbindung aber problematisch. Demgemäß kann keine über einen längeren Zeitraum lagerstabile wässrige Lösung mit einem hohen Gehalt an Magnesiumverbindung erhalten werden.

Die der vorliegenden Erfindung zugrundeliegende technische Aufgabe besteht somit darin, eine Magnesiumionen-enthaltende Lösung bereitzustellen, welche über einen langen Zeitraum lagerstabil ist, insbesondere eine hohe Magnesiumkonzentration aufweist, und kein unerwünschtes Auskristallisieren der verwendeten Magnesiumverbindung oder anderer Komponenten zeigt.

Diese Aufgabe wird durch Bereitstellen der in den Ansprüchen gekennzeichneten Ausführungsformen gelöst.

Gemäß der vorliegenden Erfindung wird eine wässrige Zusammensetzung bzw. gelartige Zusammensetzung bereitgestellt, umfassend mindestens eine Magnesiumverbindung, ausgewählt aus einem Magnesiumsalz oder einer Magnesiumkomplexverbindung, und L-Carnitin, welche mindestens 20 Gew.-% der Magnesiumverbindung und mindestens 5 Gew.-% L-Carnitin umfasst.

Die in der vorliegenden Erfindung geeigneten Magnesiumsalze bzw. Magnesiumkomplexverbindungen unterliegen grundsätzlich keiner wesentlichen Beschränkung, solange sie ausreichend gut in Wasser löslich sind. Ferner sollen die Magnesiumsalze bzw. Magnesiumkomplexverbindungen eine gute Bioverfügbarkeit und ein günstiges Freisetzungsverhalten im menschlichen und tierischen Organismus aufweisen. Derartige in Wasser gut lösliche Magnesiumsalze bzw. Magnesiumkomplexverbindungen sind einem Fachmann auf dem einschlägigen Fachgebiet bekannt. Beispielsweise kann ein Magnesiumkomplex einer Aminosäure, wie ein Magnesiumkomplex der Glutaminsäure oder der Asparaginsäure, oder Gemische davon, als Magnesiumkomplexverbindung verwendet werden. Es ist besonders bevorzugt, Magnesiumaspartathydrochlorid, insbesondere Magnesium-L-aspartat-hydrochlorid, als Magnesiumkomplexverbindung zu verwenden.

In einer Ausführungsform gemäß der vorliegenden Erfindung umfaßt die wässrige bzw. gelartige Zusammensetzung vorzugsweise 40 bis 75 Gew.-%, mehr bevorzugt 50 bis 75 Gew.-%, noch mehr bevorzugt 50 bis 70 Gew.-% der Magnesiumverbindung, ausgewählt aus einem Magnesiumsalz oder einer Magnesiumkomplexverbindung, und mindestens 5 Gew.-% L-Carnitin.

L-Carnitin bzw. 3-Hydroxy-4-(trimethylammonio)-buttersäurebetain ist eine farblose, gut wasserlösliche, hygroskopische Substanz, die ein charakteristischer Bestandteil der gestreiften Muskulatur ist und vor allem im Fettsäurestoffwechsel als Carrier für Acylgruppen durch die Mitochondrien-Membran hindurch dient.

Die erfindungsgemäße Zusammensetzung enthält L-Carnitin, da durch Zugabe von

L-Carnitin die Kristallisation von Magnesiumsalzen bzw. Magnesiumkomplexverbindungen selbst in hochkonzentrierten Lösungen wirkungsvoll verhindert oder zumindest deutlich verzögert werden kann. Besonders bevorzugt liegt der Gehalt an L-Carnitin, bezogen auf die Gesamtzusammensetzung, in einem Bereich von etwa 5 bis etwa 35 Gew.-%, wobei ein Bereich von 7 bis 30 Gew.-% am meisten bevorzugt ist.

Überraschenderweise wurde festgestellt, daß selbst bei einer sehr geringen Konzentration von L-Carnitin in der erfindungsgemäßen wässrigen Lösung die problematische Kristallisation eines Magnesiumsalzes und/oder einer Magnesiumkomplexverbindung wirkungsvoll verhindert bzw. zumindest deutlich verzögert werden kann. Beispielsweise ist es möglich, eine stabile wässrige Lösung mit 70 Gew.-% Magnesiumaspartathydrochlorid zu erhalten, wenn 5 Gew.-% L-Carnitin in der Lösung vorhanden sind. Der kristallisationshemmende Effekt läßt sich sogar noch weiter steigern, wenn in der vorstehenden Lösung 15 Gew.-% L-Carnitin vorhanden sind.

Der Gehalt der Magnesiumverbindung, ausgewählt aus einem Magnesiumsalz oder einer Magnesiumkomplexverbindung, bezogen auf die Gesamtzusammensetzung, beträgt vorzugsweise mindestens 50 Gew.-%, wobei auch bei ≥ 60 Gew.-% und sogar bei ≥ 70 Gew.-% lagerstabile wässrige Lösungen erhalten werden, die keine oder eine stark verzögerte Kristallisationsneigung zeigen, wenn ≥ 5 Gew.-%, vorzugsweise ≥ 7 Gew.-%, L-Carnitin vorhanden ist. Besonders bevorzugt liegt der Gehalt der Magnesiumverbindung, ausgewählt aus einem Magnesiumsalz oder einer Magnesiumkomplexverbindung, bezogen auf die Gesamtzusammensetzung, in einem Bereich von 50 bis 75 Gew.-%, wobei ein Gehalt in einem Bereich von 50 bis 70 Gew.-% am meisten bevorzugt ist.

Im Rahmen der vorliegenden Erfindung wurde ebenso festgestellt, daß es sogar möglich ist, eine stabile gelartige wässrige Lösung zu erhalten, die einen Gehalt an L-Carnitin von bis zu etwa 60 Gew.-% aufweist, wenn der Gehalt des Magnesiumsalzes und/oder der Magnesiumkomplexverbindung mindestens 20 Gew.-%, vorzugsweise mindestens 25 Gew.-% beträgt. In einer weiteren Ausführungsform gemäß der vorliegenden Erfindung umfaßt die wässrige Zusammensetzung somit vorzugsweise mindestens 20 Gew.-% der Magnesiumverbindung, ausgewählt aus einem Magnesiumsalz oder einer Magnesiumkomplexverbindung, und L-Carnitin in einem Bereich von 15 bis zu 60 Gew.-%. Gemäß dieser Ausführungsform beträgt der Gehalt an L-Carnitin besonders bevorzugt mindestens 20 Gew.-%.

In diesem Zusammenhang wurde äußerst überraschend festgestellt, daß in 100 g einer wässrigen Lösung, die 51 Gew.-% Magnesiumaspartathydrochlorid enthält, bis zu 150 g L-Carnitin (bei 20°C) gelöst werden können, was einem Gesamtfeststoffgehalt von über 80 Gew.-% entspricht. Dies ist insbesondere deshalb unerwartet, da die maximale Löslichkeit von L-Carnitin in 100 g Wasser (bei 20°C) 250 g beträgt, so daß in der vorstehend beschriebenen Lösung bei einer Wassermenge von etwa 49 g eine maximale Löslichkeit von etwa 121 g L-Carnitin zu erwarten gewesen wäre. Dies gilt überdies ohne Berücksichtigung der Menge an Wasser, die zur Lösung von Magnesiumaspartathydrochlorid erforderlich ist.

Ohne daran gebunden zu sein, könnte ein Erklärungsversuch für diese überraschenden Resultate eine Komplexbildung zwischen den in der Lösung vorhandenen Magnesiumionen und dem verwendeten L-Carnitin sein.

Ferner wurde überraschenderweise festgestellt, daß die Dichte einer Magnesiumenthaltenden wässrigen Lösung bei Zugabe von L-Carnitin signifikant abnimmt. Beispielsweise nimmt die Dichte einer Probelösung von 25 ml (aus 51,2 Gew.-% Magnesium-L-aspartathydrochlorid, 0,1 Gew.-% Kaliumsorbat und 48,7 Gew.-% Wasser) bei Zugabe von 10 g L-Carnitin von 1,30 g/cm³ auf 1,26 g/ cm³ ab. Dies könnte die Bildung einer neuartigen Magnesium-Komplexverbindung mit L-Carnitin als Ligand bestätigen.

Die erfindungsgemäße wässrige Zusammensetzung kann weiterhin übliche Hilfsstoffe, wie Stabilisierungsmittel, Aromastoffe, Konservierungsmittel, Arzneistoffe oder Farbstoffe, enthalten. Gemäß einer bevorzugten Ausführungsform ist in der erfindungsgemäßen wässrigen Zusammensetzung Kaliumsorbat enthalten, wobei ein Gehalt von etwa 0,05 bis 0,2 Gew.-% Kaliumsorbat besonders bevorzugt ist.

Die erfindungsgemäße wässrige Zusammensetzung kann durch einfaches Vermischen von Wasser, einer Magnesiumverbindung, ausgewählt aus einem Magnesiumsalz oder einer Magnesiumkomplexverbindung, L-Carnitin und gegebenenfalls einem oder mehreren Hilfsstoffen hergestellt werden. Es ist jedoch bevorzugt, die erfindungsgemäße wässrige Zusammensetzung durch Lösen von L-Carnitin in einer zuvor gebildeten Lösung aus Wasser, einem Magnesiumsalz oder einer Magnesiumkomplexverbindung und gegebenenfalls einem oder mehreren Hilfsstoffen herzustellen.

Die erfindungsgemäße wässrige bzw. gelartige Zusammensetzung kann auch in ein entsprechendes Sprühtrocknungsprodukt umgewandelt werden, welches durch Sprühtrocknen einer wässrigen Zusammensetzung gemäß der vorliegenden Erfindung erhalten wird.

Darüber hinaus wird gemäß der vorliegenden Erfindung die Verwendung der vorstehend beschriebenen wässrigen Zusammensetzung oder des vorstehend beschriebenen Sprühtrocknungsprodukts im Humanbereich als Arzneimittel bzw. als Nahrungsergänzungsmittel oder im Veterinärbereich als Tierfutterzusatz, insbesondere für Pferde, Geflügel, Tauben, Schweine, Rinder, Schafe und Kamele, bereitgestellt.

Die erfindungsgemäße wässrige Zusammensetzung eignet sich besonders gut zur Verabreichung an Pferde oder Kamele durch einen sogenannten Maulinjektor. Durch Verwendung der erfindungsgemäßen Zusammensetzung kann das unerwünschte Blockieren des Kolbens in einem Maulinjektor durch Auskristallisieren der enthaltenen Magnesiumverbindung wirkungsvoll verhindert werden.

Das in der erfindungsgemäßen Zusammensetzung eingesetzte L-Carnitin weist somit nicht nur den überraschenden Effekt der Kristallisationshemmung bzw. Stabilisierung der wässrigen Lösung auf, sondern bietet in seiner Eigenschaft, den Stoffwechsel, insbesondere im Muskelgewebe, und den Muskelaufbau bzw. die Leistungssteigerung bei Mensch und Tier zu unterstützen, beim Einsatz der erfindungsgemäßen Zusammensetzung als Arzneimittel, Nahrungsergänzungsmittel oder als Tierfutterzusatz weitere vorteilhafte Eigenschaften.

Die nachfolgenden Beispiele werden angegeben, um die vorliegenden Erfindung weiter zu erläutern, ohne diese darauf zu beschränken.

### Beispiele

### Beispiel 1 (insbesondere als Veterinärprodukt)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
43,35 g Magnesium-L-aspartathydrochlorid
0,05 g Kaliumsorbat
30,24 g Wasser
26,36 g L-Carnitin

### Beispiel 2 (insbesondere als Veterinärprodukt)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
49,84 g Magnesium-L-aspartathydrochlorid
0,05 g Kaliumsorbat
20,00 g Wasser
30,10 g L-Carnitin

### Beispiel 3 (insbesondere als Veterinärprodukt)

Eine lagerstabile wässrige Lösung, die keine Kristallisationsneigung bei Lagerung über mehrere Wochen bei 20°C zeigte, wurde erhalten, wenn die nachfolgenden Bestandteile vermischt wurden:
28,13 g Magnesium-L-aspartathydrochlorid
0,029 g Kaliumsorbat
54,86 g Wasser
16,99 g L-Carnitin

### Beispiel 4 (insbesondere als Humanprodukt)

Ein lagerstabiles wässriges Gel, das keine Kristallisationsneigung bei Lagerung über mehrere Wochen bei 20°C zeigte, wurde erhalten, wenn die nachfolgenden Bestandteile vermischt wurden:
60,51 g Magnesium-L-aspartathydrochlorid
0,07 g Kaliumsorbat
29,93 g Wasser
9,49 g L-Carnitin

### Beispiel 5 insbesondere als Humanprodukt)

Eine lagerstabile wässrige Lösung, die keine Kristallisationsneigung bei Lagerung über mehrere Wochen bei 20°C zeigte, wurde erhalten, wenn die nachfolgenden Bestandteile vermischt wurden:
43,22 g Magnesium-L-aspartathydrochlorid
0,07 g Kaliumsorbat
49,93 g Wasser
6,78 g L-Carnitin

### Beispiel 6

Eine lagerstabile wässrige gelartige Lösung, die keine Kristallisationsneigung bei Lagerung über mehrere Wochen bei 20°C zeigte, wurde erhalten, wenn die nachfolgenden Bestandteile vermischt wurden:
51,2 g Magnesium-L-aspartathydrochlorid
0,1 g Kaliumsorbat
48,7 g Wasser
100 g L-Carnitin

### Beispiel 7

Eine lagerstabile wässrige gelartige Lösung, die keine Kristallisationsneigung bei Lagerung über mehrere Wochen bei 20°C zeigte, wurde erhalten, wenn die nachfolgenden Bestandteile vermischt wurden:
51,2 g Magnesium-L-aspartathydrochlorid
0,1 g Kaliumsorbat
48,7 g Wasser
150 g L-Carnitin

### Vergleichsbeispiel 1

Eine nicht lagerstabile wässrige Lösung, die nach wenigen Tagen einen kristallinen Niederschlag von Magnesium-L-aspartathydrochlorid zeigte, wurde erhalten, wenn die nachfolgenden Bestandteile vermischt wurden:
70 g Magnesium-L-aspartathydrochlorid
0,1 g Kaliumsorbat
30 g Wasser

## Patentansprüche

1. Wässrige bzw. gelartige Zusammensetzung, umfassend mindestens 20 Gew.-% der Magnesiumkomplexverbindung Magnesium-L-aspartat-Hydrochlorid und L-Carnitin in einem Bereich von 15 bis zu 60 Gew.-%.

2. Zusammensetzung nach Anspruch 1, umfassend mindestens 20 Gew.-% L-Carnitin.

3. Zusammensetzung nach Anspruch 1 oder 2, umfassend mindestens 25 Gew.-% der Magnesiumverbindung.

4. Zusammensetzung nach einem der Ansprüche 1 bis 3, weiter umfassend ein oder mehrere Hilfsstoffe.

5. Verwendung einer Zusammensetzung nach einem der Ansprüche 1 bis 4 als Nahrungsergänzungsmittel oder als Tierfutterzusatz.

6. Verwendung einer Zusammensetzung nach einem der Ansprüche 1 bis 4 zur Herstellung eines Arzneimittels zur Unterstützung des Stoffwechsels, insbesondere im Muskelgewebe, und des Muskelaufbaus.

## Claims

1. An aqueous or gel-like composition comprising at least 20 wt.-% of the magnesium complex compound magnesium-L-aspartate-hydrochloride and L-carnitine in an amount of 15 up to 60 wt.-%.

2. The composition according to claim 1 comprising at least 20 wt.-% L-carnitine.

3. The composition according to claim 1 or 2 comprising at least 25 wt.-% of the magnesium compound.

4. The composition according to any one of claims 1 to 3, further comprising one or more auxiliaries.

5. Use of a composition according to any one of claims 1 to 4 as food supplement, or as animal feed additive.

6. Use of a composition according to any one of claims 1 to 4 for the preparation of a medicament for supporting metabolism, in particular in muscle tissue, and for supporting muscle structure.

## Revendications

1. Composition aqueuse ou de type gel, comprenant au moins 20 % en poids du composé de complexe de magnésium, chlorhydrate de L-aspartatemagnésium et de la L-carnitine dans une plage de 15 jusqu'à 60 % en poids.

2. Composition selon la revendication 1, comprenant au moins 20% en poids de la L-carnitine.

3. Composition selon la revendication 1 ou 2, comprenant au moins 25% en poids du composé de magnésium.

4. Composition selon l'une des revendications 1 à 3, comprenant en outre un ou plusieurs adjuvants.

5. Utilisation d'une composition selon l'une des revendications 1 à 4, comme complément alimentaire ou comme additif alimentaire pour animaux.

6. Utilisation d'une composition selon l'une des revendications 1 à 4, pour la production d'un médicament pour stimuler le métabolisme, en particulier dans les tissus musculaires et pour la constitution musculaire.
